# EUROPÄISCHE PATENTANMELDUNG

(11) **EP 1 178 315 A1**
(43) Veröffentlichungstag der Anmeldung: **06.02.2002**
(21) Anmeldenummer: 00116515.8
(22) Anmeldetag: 31.07.2000
(51) Int. Cl.: G01N 33/487, C12M 1/34

(54) **Verfahren und Vorrichtung zur Untersuchung von Zellen mit Hilfe der Patch Clamp-Methode**

(71) Anmelder: Lepple-Wienhues, Albrecht, Dr.med., Priv.Doz., 72070 Tübingen (DE)
(72) Erfinder: Lepple-Wienhues, Albrecht, Dr.med., Priv.Doz., 72070 Tübingen (DE)
(74) Vertreter: Patentanwälte Ruff, Wilhelm, Beier, Dauster & Partner

(57) **Zusammenfassung**

Bei einem Verfahren zur Untersuchung von Zellen oder dergleichen mit Hilfe der Patch Clamp-Methode wird mindestens eine Zelle in das Innere einer Kapillare eingebracht und dort unter Ausbildung eines ausreichend dichten Kontaktes zwischen Zellmembran und Kapillarinnenfläche positioniert. Die Kapillare weist dabei auf ihrer Länge mindestens eine Verengung mit einem Durchmesser, der kleiner als der Durchmesser der Zelle ist, auf. Vorzugsweise können die Zellen in der Kapillare durch Einspülen oder Einsaugen oder Einzentrifugieren eingebracht und positioniert werden. Weiter ist eine Vorrichtung zur Durchführung des erfindungsgemäßen Verfahrens offenbart.

## Beschreibung

Die Erfindung betrifft ein Verfahren zur Untersuchung von Zellen oder dergleichen mit Hilfe der Patch Clamp-Methode sowie eine entsprechende Vorrichtung.

Mit Hilfe von Lipidmembranen stellen lebende Zellen in ihrem Inneren eine Mikroumgebung her, die die Funktion des zellulären Apparates, insbesondere des zellulären Enzymapparates sichert. Solche Lipidmembranen sind praktisch undurchlässig für geladene Teilchen. Deshalb transportieren spezialisierte Transportproteine in der Zellmembran die Ionen und stellen damit die Grundlage für eine Vielzahl physiologischer Funktionen dar, darunter alle elektrischen Erregungsvorgänge im Organismus, Stofftransport durch Membranen oder Zellschichten, sowie eine Reihe von zellulären elektrischen und chemischen Signalen.

Im obigen Zusammenhang hat die Patch Clamp-Methode seit ihrer Entdeckung im Jahr 1981 durch Sakmann und Neher die Zellbiologie revolutioniert. Die Patch Clamp-Technik erlaubt es, Ströme durch Ionentransporter direkt mit hoher zeitlicher Auflösung zu messen und somit ein Membranprotein gewissermaßen bei seinen Konformationsänderungen zu "belauschen". In der Tat ist somit die Patch Clamp-Methode das einzige Verfahren, das es gestattet, Konformationsänderungen einzelner spezialisierter Proteinmoleküle direkt mit einer hohen zeitlichen Auflösung im Bereich weniger ms zu beobachten. Dies ermöglicht es beispielsweise, die Wirkung von Signalmolekülen oder von pharmakologischen Substanzen direkt am Zielprotein zu messen. Darüberhinaus ermöglicht es diese Technik, die Umgebung der Membran elektrisch und chemisch exakt zu kontrollieren und sowohl auf die Innen- als auch die Außenseite der Membran Signalstoffe, Medikamente und dergleichen aufzubringen.

Zu den Transportproteinen, die mit dieser Technik untersucht werden können, zählen elektrogene Ionentransporter für organische und anorganische Ionen, selektive und nichtselektive Ionenkanäle und andere porenbildende Membranproteine. Zu den Ionenkanälen zählen spannungsgesteuerte, ligandengesteuerte und speichergesteuerte Kanäle, die mehr oder weniger selektiv für Na⁺, K⁺, Ca²⁺, und Cl⁻ sind. Selbst elektrische Ladungsverschiebungen innerhalb eines membranassoziierten Proteins können mit der Patch Clamp-Technik gemessen werden (gating charges), wodurch alle membranassoziierten Proteine wie Rezeptoren und Enzyme und deren Interaktion mit anderen Proteinen oder geladenen Molekülen mit der Technik untersucht werden können. Ionenkanäle sind gewebespezifisch verteilt und zeigen eine große funktionelle Vielfalt. Sie sind daher ideale Zielmoleküle für pharmakologische Substanzen, da organspezifische Funktionen gezielt beeinflußt werden können. Beispiele für solche Substanzen sind Diuretika, Antidiabetika, Antiepileptika, Lokalanästhetika, Antiarrhytmika, einige Antibiotika etc. Die Patch Clamp-Methode stellt eine hochsensitive Meßmethode für die Wirkung solcher Substanzen dar. Der größte Nachteil dieser Methode besteht in ihrer Kompliziertheit, der erforderlichen manuellen Manipulationen und dem daraus bedingten geringen Durchsatz von zu testenden Substanzen und Transportproteinen.

Die Durchführung der Patch Clamp-Methode ist nach unterschiedlichen Varianten bekannt. Allen diesen Varianten ist es jedoch gemeinsam, daß üblicherweise Zellen mit einer Mikropipette aus Glas unter mikroskopischer Sichtkontrolle mechanisch aufgesucht und angesaugt werden. Auf diese Weise wird eine elektrisch dichte innige Verbindung der Zellmembran mit der Pipettenspitze hergestellt, was die rauscharme hochauflösende Messung von kleinsten Strömen erlaubt. Je nach Variante werden die Ströme durch die unter der Pipettenöffnung befindliche Membran oder durch die gegenüberliegende Membran aufgezeichnet.

Im Zusammenhang mit der dichten Verbindung der Zellmembran mit der Pipettenspitze spricht man oft auch von einer "Verklebung" der Zellmembran mit der entsprechenden Fläche der Glaskapillare. Ein sogenannter "Gigaseal" ist dann erreicht, wenn (nach Annäherung der unter Unterdruck gesetzten Kapillare an die Zellmembran und Ausbildung der Verklebung) ein elektrischer Widerstand im Gigaohm-Bereich entsteht. Dieser üblicherweise entstehende Widerstand von einigen 10 GOhm zeigt, daß selbst kleinste Ionen wie Protonen nicht mehr zwischen Membran und Glasrand passieren können. Eine mit der Technik vertraute Person ging bisher davon aus, daß für die Verklebung der Zellmembran mit der Glaspipette der Rand der Pipettenöffnung entscheidend ist, auf den die Zelle durch mechanischen Druck und Ansaugen aufgebracht wird. Aufgrund dieser Tatsache können dann mit Hilfe moderner Meßverstärker Gleichströme im Bereich bis ca. 100 fA (Femtoampere), also in der Größenordnung von 10⁻¹³ A gemessen werden. In einigen Fällen genügt auch eine geringere Dichtigkeit der Verbindung von Zelle und Meßapparatur, wenn die zu untersuchenden Ströme sehr groß sind ("loose patch"). Die gemessenen Gleichströme repräsentieren in jedem Fall die Summe aus Ionentransport durch die Zellmembran und Leckströme in der Meßapparatur.

Insbesondere aufgrund des beschriebenen Prinzips der mechanischen Annäherung der Mikropipette an die Zellmembran ist die Patch Clamp-Methode überaus personal- und zeitintensiv. Die Ansteuerung jeder einzelnen Zelle mit der Mikropipette unter mikroskopischer Sichtkontrolle und die Ansaugung dieser Zelle erfordert nicht nur großes manuelles Geschick, sondern auch eine genaue Kenntnis der mechanischen Eigenschaften der untersuchten Zelle und ihrer Mikromanipulation. So ist beispielsweise der angewendete Druck der Mikropipette auf die Zelle kritisch für die Erzielung der innigen Verbindung zwischen Membran und Pipettenspitze. Ferner ist die erzielte innige Verbindung mechanisch labil und wird durch Erschütterung oder Flüssigkeitsturbulenzen (z. B. bei Wechsel einer Lösung) häufig zerstört. Ferner werden üblicherweise ein Mikroskop und ein Mikromanipulator benötigt, die zu einem erheblichen Raumbedarf für die Vorrichtung insgesamt führen und damit sowohl einer Miniaturisierung als auch einer grundsätzlich erwünschten Mehrfachanordnung der Mikropipetten entgegenstehen.

Die obigen Ausführungen zeigen, daß sich der Patch Clamp-Methode eine Vielzahl von weiteren Einsatzmöglichkeiten erschließen würden, wenn es gelingen würde, eine Vielzahl von Zellen gleichzeitig zu untersuchen und die Erstellung des Gigaseals zu automatisieren. Dem steht jedoch bei den bisherigen konventionellen Methoden im Weg, daß die Öffnung der Mikropipette mit der Zellmembran mechanisch in Berührung gebracht werden muß. Die DE-A1-197 44 649 offenbart zwar eine Vorrichtung und ein Verfahren, bei dem gleichzeitig mit Hilfe einer Vielzahl von Mikropipetten eine Vielzahl von Zellen untersucht werden soll. Jedoch macht auch diese Druckschrift von dem oben genannten Grundprinzip Gebrauch, daß die Mikropipetten in Richtung auf die Zellmembran mechanisch zubewegt werden. Die WO 99/66329 beschreibt eine Anordnung von Poren in einem dünnen flächigen Substrat, auf die Zellen aufgebracht werden, um eine Vielzahl von Zellen gleichzeitig zu untersuchen. Die Herstellung eines solchen porösen Substrates ist aufwendig und teuer und die Verklebung von mehreren Zellen mit einer Vielzahl Poren ist zufällig und aufwendig zu realisieren sowie zu kontrollieren. Die Verklebung von Zellen mit der Oberfläche eines porösen Substrats ist ferner mechanisch labil und durch Lösungswechsel leicht zu zerstören. Außerdem wird bei einer flächigen Anordnung von Poren wie in WO 99/66329 sowie bei ähnlichen dem Fachmann bekannten transepithelialen Messvorrichtungen (Ussing-Kammern) die Messung bereits dann gestört, wen einzelne dieser Poren nicht ausreichend dicht mit einer Zelle verklebt sind. Es treten durch undichte Stellen erhebliche Leckströme auf, die mehrere Größenordnungen über den zu messenden Strömen durch Ionentransporter liegen. Die große elektrische Kapazität solcher Vorrichtungen bedingt ferner starke elektrische Störungen und reduziert die zeitliche Auflösung der Messung, die besonders bei spannungsgesteuerten lonenkanälen kritisch ist.

Die Erfindung stellt sich demgegenüber die Aufgabe, ein Verfahren und eine Vorrichtung zur Verfügung zu stellen, bei dem die Nachteile des Standes der Technik vermieden werden. Insbesondere soll ein neuer Ansatz gefunden werden, um die für die Patch Clamp-Methode notwendige elektrische Abdichtung der Zellmembran zu erreichen. Dabei soll die Verbindung der Zellmembran mit dem entsprechenden abdichtenden Material mechanisch stabiler werden. Die zwingende Notwendigkeit einer visuellen bzw. mikroskopischen Kontrolle des Abdichtvorganges soll entfallen. Die Erfindung soll eine möglichst geringe elektrische Kapazität der Meßanordnung und daraus folgend geringe dielektrische Fluktuationen (Rauschen) und eine hohe zeitliche Auflösung des Meßsignals gewährleisten. Insbesondere soll es die Erfindung ermöglichen, daß eine Vielzahl von Zellen entweder gleichzeitig oder doch in rascher zeitlicher Abfolge mit Hilfe der Patch Clamp-Methode untersucht werden kann. Dabei soll eine solche möglicherweise automatisierbare Vorrichtung möglichst mit einfach produzierbaren oder bereits bekannten Bauteilen herstellbar sein. Neben einer Automatisierung wird auch eine Miniaturisierung der Vorrichtung angestrebt, um die Vorrichtung beispielsweise in marktgängige Laborrobotersysteme zu integrieren.

Die geschilderten und weitere Aufgaben werden durch das Verfahren mit den Merkmalen des Anspruchs 1 bzw. durch die Vorrichtung mit den Merkmalen des Anspruchs 8 gelöst. Bevorzugte Ausführungen des Verfahrens bzw. der Vorrichtung sind in den abhängigen Ansprüchen 2 bis 7 bzw. 9 bis 17 dargestellt. Der Wortlaut sämtlicher Ansprüche wird hiermit durch Bezugnahme zum Inhalt dieser Beschreibung gemacht.

Das erfindungsgemäße Verfahren zeichnet sich dadurch aus, daß die zu untersuchende Zelle in das Innere einer Kapillare eingebracht und dort unter Ausbildung eines ausreichend dichten Kontaktes zwischen Zellmembran und Kapillarinnenfläche, welcher für die Durchführung der Patch Clamp-Untersuchung erforderlich ist, positioniert wird. Anschließend kann an der derart positionierten Zelle die Patch Clamp-Untersuchung vorgenommen werden. Die bei diesem Verfahren verwendete Kapillare weist auf ihrer Länge mindestens eine Verengung auf, wobei diese Verengung einen Durchmesser besitzt, der kleiner ist als der Durchmesser der zu untersuchenden Zelle.

Unter Verengung oder Engstelle im Sinne der Erfindung soll dabei jede Ausgestaltung der Kapillare verstanden werden, die zum einen die in die Kapillare eingebrachte Zelle im Inneren der Kapillare festhält und somit ein Hindurchtreten der Zelle durch die Kapillare verhindert und zum anderen die Zelle auf einer im wesentlichen geschlossenen Fläche zur Ausbildung des dichten Kontaktes umschließen kann. Dementsprechend wird es sich bei der Verwendung von Kapillaren mit kreisförmigem Querschnitt bei der Verengung üblicherweise um eine ringförmige Verengung oder Engstelle handeln, an der die Zelle festgehalten und dichtend positioniert wird. Selbstverständlich kann der Durchmesser der Verengung variiert werden, um auf diese Weise eine Anpassung an den Durchmesser der zu untersuchenden Zellen vorzunehmen. Durch Verwendung entsprechend kleiner Kapillarquerschnitte können auch sehr kleine Zellen oder subzelluläre Membranstrukturen wie z. B. Mitochondrien, Lysosomen oder Bakterien positioniert werden. Diese Strukturen waren bisher mit der herkömmlichen Patch Clamp-Tehnik nicht direkt untersuchbar, da sie für die mikromechanische Manipulation und lichtmikroskopische Kontrolle zu klein sind. Daher ist in dieser Anmeldung der Begriff "Zelle" im Sinne einer biologischen Struktur zu verstehen, die mit einer Lipidmembran umgeben ist.

Unter Positionierung im Sinne der Erfindung soll die für die Patch Clamp-Untersuchung ausreichende Fixierung der Zelle an der Verengung innerhalb der Kapillare verstanden werden, wobei ein elektrisch ausreichend dichter Kontakt zwischen Zellmembran und der Kapillarinnenfläche hergestellt ist. Die Kontaktfläche zwischen Zellmembran und Kapillarinnenfläche ist dabei so ausreichend geschlossen, daß die Patch Clamp-Untersuchung vorgenommen werden kann.

Die Länge der Kapillare ist bei dem erfindungsgemäßen Verfahren nicht kritisch, so daß grundsätzlich Kapillaren ganz unterschiedlicher Länge eingesetzt werden können. Sinnvollerweise wird die Kapillare zumindest so lang sein, daß eine zu untersuchende und an der Verengung festgehaltene Zelle sich zumindest vollständig innerhalb der Kapillare befindet.

Aus den bisherigen Ausführungen geht hervor, daß die Verengung bei dem erfindungsgemäßen Verfahren auf unterschiedliche Weise ausgebildet sein kann. Bei einer vergleichsweise einfachen Konstruktion handelt es sich bei der Verengung um eine Verjüngung innerhalb der Kapillare, d. h. um einen Bereich der Kapillare, in dem der Durchmesser der Kapillare entweder sprungförmig oder vorzugsweise allmählich abnimmt. In solchen Fällen wird dann die zu untersuchende Zelle im Bereich der Verjüngung dort fixiert, wo der Durchmesser der Kapillare im wesentlichen dem Durchmesser der Zelle entspricht. Insbesondere kann die Verjüngung so ausgestaltet sein, daß die Zelle im Bereich der Austrittsöffnung, d. h. auf der der Einbringseite gegenüberliegenden Seite der Kapillare, festgehalten und positioniert wird.

Bei der Kapillare mit einer als Verjüngung ausgebildeten Verengung kann es sich vorzugsweise um eine sogenannte Mikropipette handeln, wie sie auch jetzt bereits in verschiedener Ausführung für die Patch Clamp-Methode verwendet werden. Die Ausgestaltung und Herstellung solcher Mikropipetten ist dem Fachmann bekannt. Solche Mikropipetten werden typischerweise durch lokales Erhitzen und Schmelzen von Glaskapillaren mit anschließendem Auseinanderziehen der Kapillare gefertigt. Grundsätzlich können solche Mikropipetten bei dem erfindungsgemäßen Verfahren aus unterschiedlichen (im wesentlichen nichtleitenden) Materialien gefertigt sein (z. B. Kunststoffe wie Polystyrol). Es ist jedoch bevorzugt, wenn bei der Erfindung Glaskapillaren, vorzugsweise Mikropipetten aus Glas eingesetzt werden. Glas ist ein bewährtes Material für die dichte Verklebung mit Membranen und hat gute dielektrische Eigenschaften. Glas ist weitgehend chemisch inert und kann auch vergleichsweise einfach gereinigt werden. Außerdem können aus Glas in Standard-Glasbläsertechnik leicht die notwendigen Kapillaren, insbesondere Mikropipetten in einem weiten Bereich von Öffnungsdurchmessern kostengünstig hergestellt werden. Mikropipetten mit Öffnungsdurchmessern zwischen 10 µm und 50 nm je nach Größe der zu untersuchenden Struktur werden vorzugsweise verwendet.

Bei bevorzugten Ausführungen des erfindungsgemäßen Verfahrens erfolgt das Einbringen und Positionieren der Zelle in die Kapillare durch Einspülen oder Einsaugen einer Suspension oder Lösung, die die Zelle enthält. Bei diesen Ausführungen wird beispielsweise die Zellsuspension in die (große) Öffnung der Mikropipette eingespült, so daß mindestens eine der Zellen in den sich verjüngenden Teil der Mikropipette eingebracht wird. Dort wird bereits durch das Einspülen/Einsaugen selbst die Positionierung bewirkt, d. h. die innige Verbindung der Zelle mit der Innenseite der Mikropipette im Bereich der Verjüngung. Durch die damit verbundene Blockierung der Kapillare kommt der Flüssigkeitsstrom zum Erliegen, so daß keine weiteren Zellen an die Verengung/Engstelle gelangen können. Auf diese Weise erfolgt das Einbringen und das Positionieren quasi in einem Verfahrensschritt.

Alternativ oder zusätzlich kann bei weiteren Ausführungen des erfindungsgemäßen Verfahrens das Einbringen und Positionieren der Zelle in die Kapillare durch Einzentrifugieren einer Suspension oder Lösung, die die Zelle enthält, erfolgen. Auf diese Weise wird beispielsweise die Zellsuspension in eine Mikropipette eingeschleudert, wobei dann auch hier die Positionierung in der bereits beschriebenen Weise erfolgt.

Alternativ oder zusätzlich kann bei weiteren Ausführungen des erfindungsgemäßen Verfahrens das Einbringen und Positionieren der Zelle durch mechanische Vibrationen oder durch das Anlegen eines elektrischen Feldes längs der Pipette erleichtert werden. Eine weitere Möglichkeit, Zellen einzubringen und zu positionieren, besteht darin, daß magnetische beads an die Zelle gekoppelt und die Zelle mit Hilfe von Magnetfeldern positioniert wird oder daß die Zelle mit Hilfe von Laserlicht (optical tweezers) positioniert wird.

Das erfindungsgemäße Verfahren kann weiter dadurch gekennzeichnet sein, daß an der positionierten Zelle oder dergleichen Messungen des Gleichstroms in der Spannungsklemme, des Potentials in der Stromklemme, des elektrischen Widerstandes, der Impedanz, der elektrischen Kapazität, der Fluoreszenz, der Plasmoresonanz, der mechanischen Resonanz, der Fluidität, der Rigidität vorgenommen werden.

Gegebenenfalls kann die Positionierung der Zelle im Kapillarinneren vor Durchführung der eigentlichen Patch Clamp-Untersuchung kontrolliert werden. Dies kann beispielsweise dadurch erfolgen, daß optische Signale, beispielsweise aus einer Beleuchtung der Verengung der Kapillare, insbesondere mit Laserlicht, ausgewertet werden. In diesem Zusammenhang ist es grundsätzlich ebenfalls möglich, die zu untersuchenden Zellen mit Farbstoffen, farbstoffgekoppelten Antikörpern, Liganden, Lipiden etc. einzufärben. Diese Färbung kann gegebenenfalls auch chemische Veränderungen im Zellinneren oder auf der Zelloberfläche anzeigen, so daß sich eine solche Messung nicht nur auf die Feststellung der Position der Zelle im Kapillarinneren beschränkt, sondern auch solche chemischen Veränderungen im Zellinneren erfassen kann. Vorzugsweise wird die Positionierung der Zelle durch eine Druckmessung oder Durchflußmessung (Messung der Durchflußvolumina) beim Einspülen/Einsaugen/Einzentrifugieren der Zellen gemessen. Eine entsprechende Druckänderung oder eine entsprechende Reduzierung des Durchflusses zeigt an, daß mindestens eine Zelle im Kapillarinneren positioniert ist. Dadurch ist es auch möglich, Drücke und Durchflußvolumina zu regulieren, insbesondere automatisch zu regulieren, um auf diese Weise eine gewünschte Positionierung der Zelle im Kapillarinneren, d. h. eine innige Verbindung mit dem Kapillarinneren zu erreichen. Schließlich kann die Positionierung der Zelle vorzugsweise auch durch Messung des elektrischen Widerstands in der Kapillare gemessen werden, um auf diese Weise die Qualität der Verbindung der Zelle mit dem Kapillarinneren zu beurteilen und gegebenenfalls zu regulieren. Durch Regulieren des Einspüldruckes bzw. der Zentrifugationsgeschwindigkeit abhängig vom elektrischen Widerstand kann für verschiedene Zellarten eine optimale Verklebung mit der Innenwand der Pipette erzielt werden.

Bei dem erfindungsgemäßen Verfahren ist es grundsätzlich möglich, daß die Kapillare, insbesondere die Mikropipette nach erfolgter Patch Clamp-Untersuchung ausgewechselt wird. Auf diese Weise steht für eine neue Messung wieder eine (hochreine) Oberfläche für die Fixierung der dann zu untersuchenden Zellen zur Verfügung. Erfindungsgemäß ist es jedoch bevorzugt, wenn die untersuchte Zelle nach Durchführung der Patch Clamp-Untersuchung aus der Kapillare wieder entfernt wird. Dies kann in einfacher Weise durch ein Ausspülen oder Aussaugen der Zelle erfolgen. Danach wird die Kapillare gereinigt und kann dann für eine weitere Patch Clamp-Untersuchung durch Fixierung neuer Zellen vorbereitet werden. Die Reinigung der Kapillare erfolgt dabei vorzugsweise durch Spülung mit geeigneten Lösungsmitteln und/oder Chemikalien, wobei alternativ oder zusätzlich Wärme/Hitze und/oder Ultraschall zum Reinigen angewendet werden kann.

Es versteht sich von selbst, daß das beanspruchte Verfahren auf beliebige Weise modifiziert werden kann. So kann vorgesehen sein, daß auf beiden Seiten der in der Kapillare verklebten Zelle Lösungen mit pharmakologischen oder biologisch wirksamen Substanzen an die Zellmembran gebracht werden. Dies kann beispielsweise mit Hilfe der ohnehin zum Einbringen der Zelle oder zum Reinigen der Kapillare vorhandenen Spülvorrichtungen oder dergleichen geschehen. Weiterhin ist es möglich, daß die Zellmembran der verklebten Zelle selektiv an einer Stelle/auf einer Seite durchlässig gemacht wird. Dazu können entweder geeignete Druckpulse, geeignete Spannungspulse oder auch membranpermeabilisierende Substanzen (z. B. Ionophore, Tenside, Enzyme) eingesetzt werden. Auch hierfür können die ohnehin vorhandenen Einrichtungen genutzt werden. Durch dieses "Durchlässigmachen" wird eine beliebige Seite der Zellmembran beispielsweise für einseitig in die Kapillare eingebrachte Substanzen zugänglich gemacht und der elektrische Widerstand für diesen Zugang verringert.

Es ist offensichtlich, daß mit Hilfe des erfindungsgemäßen Verfahrens die unterschiedlichsten Zellen untersucht werden können. Neben üblichen Zellen aus Zellkulturen wie z. B. Jurkat Lymphomzellen, HEK293 cells, Chinese hamster ovary (CHO) Zellen, primäre Zellen aus neuronalem Gewebe wie Hippocampus, Hinterwurzelganglion, Neuroendokrine Zellen aus Nebennierenmark etc.; Skelettmuskel; Glatter Muskel; Herzmuskel, Zellen des Immunsystems; Epithelien und Endothelien etc. können auch Zellen untersucht werden, die gentechnisch verändert wurden, beispielsweise solche, bei denen Ionentransportproteine künstlich in der Membran exprimiert werden, zum Beispiel genetisch veränderte Transportproteine in CHO Zellen. Bei solchen Ausführungen kann sich an die Patch Clamp-Untersuchung auch ein Verfahrensschritt anschließen, bei dem genetisches Material aus den untersuchten Zellen gewonnen wird. Schließlich wird darauf hingewiesen, daß beispielsweise auch Lipidvesikel nach dem erfindungsgemäßen Verfahren in das Kapillarinnere eingebracht werden können und damit das entsprechende erfindungsgemäße Verfahren durchgeführt werden kann. Vorzugsweise sind in solche Lipidvesikel Transportproteine, gegebenenfalls genetisch verändert, eingebracht. Auoßerdem können subzelluläre Membranstrukturen (z. B. Mitochondrien, Lysosomen, Endoplasmatisches Retikulum, Zellkerne) oder Pflanzenzellen sowie Prokaryonten (z. B. Bakterien) untersucht werden, da im Gegensatz zum herkömmlichen Patch Clamp-Verfahren mit dem erfindungsgemäßen Verfahren auch Strukturen unter 1 µM Durchmesser positioniert werden können.

Das erfindungsgemäße Verfahren umfaßt auch die Möglichkeit, daß an der positionierten Zelle Proteine, Ribonukleinsäuren, Desoxyribonukleinsäuren, Enzyme und andere Moleküle aus dieser Zelle entnommen werden.

Weiter umfaßt die Erfindung eine Vorrichtung zur Untersuchung von Zellen oder dergleichen mit Hilfe der Patch Clamp-Methode, die insbesondere zur Durchführung des erfindungsgemäßen Verfahrens vorgesehen ist. Diese Vorrichtung umfaßt mindestens eine Kapillare, mindestens eine Einrichtung zum Einbringen einer Zelle in das Kapillarinnere und zur Positionierung der Zelle im Kapillarinneren, und gegebenenfalls weitere übliche Bauteile einer Vorrichtung zur Durchführung der Patch Clamp-Methode. Die genannte Kapillare zeichnet sich dadurch aus, daß sie auf ihrer Länge mindestens eine Verengung aufweist, wobei die Verengung einen Durchmesser besitzt, der kleiner ist als der Durchmesser einer zu untersuchenden Zelle. Weiter ist die Kapillare dazu vorgesehen, daß eine Zelle in ihr Inneres eingebracht und dort unter Ausbildung eines ausreichend dichten Kontaktes zwischen Zellmembran und Kapillarinnenfläche positioniert wird. Bezüglich der besonderen Merkmale dieser Vorrichtung und der damit verbundenen Vorteile wird insbesondere auch auf die obige Beschreibung verwiesen und ausdrücklich Bezug genommen.

Bei bevorzugten Ausführungsformen der erfindungsgemäßen Vorrichtung ist die Einbring- und Positioniereinrichtung zum Einspülen/Einsaugen mindestens einer Suspension oder Lösung in das Kapillarinnere ausgebildet. Zu diesem Zweck können insbesondere Behältnisse zur Aufnahme der Suspensionen/ Lösungen, Zuleitungen und/oder Ableitungen zwischen den Behältnissen und der Kapillare, entsprechende Pumpeinrichtungen für die Suspensionen/Lösungen sowie gegebenenfalls Meßeinrichtungen (Druck, Durchflußvolumina) vorgesehen sein.

Bei anderen Ausführungsformen ist die Einbring- und Positioniereinrichtung zusätzlich oder alternativ zum Einzentrifugieren mindestens einer Suspension/Lösung ausgebildet.

In Weiterbildung können Einrichtungen zur Messung des elektrischen Widerstandes oder Einrichtungen zur Auswertung von optischen Signalen, vorzugsweise Laserlichtsignalen vorgesehen sein. Diese können dazu dienen, die Positionierung der Zelle im Kapillarinneren zu kontrollieren und gegebenenfalls zu regulieren.

Die Vorteile der Erfindung zeigen sich in besonderer Weise bei bevorzugten Ausführungen der erfindungsgemäßen Vorrichtung, bei denen eine Vielzahl von eine entsprechende Verengung aufweisenden Kapillaren, insbesondere von Mikropipetten vorgesehen sind. Die Kapillaren sind dabei vorzugsweise in einem regelmäßigen Muster flächig angeordnet. Auf diese Weise läßt sich die Patch Clamp-Methode automatisieren, da entweder gleichzeitig oder doch zumindest in rascher zeitlicher Abfolge Zellen, die in den Kapillaren fixiert sind, untersucht werden können.

Bei den Ausführungen mit der Vielzahl von Kapillaren können in einem ersten Schritt die Kapillaren mit Zellen oder dergleichen bestückt werden. Da die Zelle in der Kapillare mechanisch geschützt und stabil liegt, können in einem zweiten Schritt Kapillaren mit ausreichend dichter Verklebung der Membran in die Meßvorrichtung überführt werden.

Bei den Ausführungen mit einer Vielzahl von Kapillaren sind vorzugsweise auch eine Vielzahl von Behältnissen, insbesondere sogenannte Mikroküvetten vorhanden, die zur Aufnahme der Suspensionen oder Lösungen mit den Zellen oder dergleichen dienen. Bei den Behältnissen kann es sich auch um Vertiefungen einer Mikrotiterplatte handeln. Vorzugsweise entspricht die Anzahl an Behältnissen der Anzahl an Kapillaren, so daß eine definierte Suspension aus einem Behältnis in eine definierte Kapillare überführt werden kann.

Bei den beschriebenen Ausführungen sind die Kapillaren und die Behältnisse vorzugsweise so zueinander angeordnet oder zueinander beweglich, daß die Suspensionen/Lösungen und damit die in ihnen enthaltenen Zellen in das Innere der Kapillaren überführbar sind. Im Zusammenhang mit diesen Ausführungen wird auch auf die Figurenbeschreibung verwiesen. Schließlich umfaßt die Erfindung die Verwendung einer Kapillare, die auf ihrer Länge mindestens eine Verengung mit einem Durchmesser, der kleiner als der Durchmesser einer Zelle ist, aufweist, zur Durchführung des erfindungsgemäßen Verfahrens oder bei einer erfindungsgemäßen Vorrichtung. Die Kapillare ist vorzugsweise aus Glas gefertigt, wobei es sich insbesondere um eine sogenannte Mikropipette handelt.

Die beschriebenen Merkmale und weitere Merkmale der Erfindung ergeben sich aus der nachfolgenden Beschreibung eines Versuches und der Figuren. Hierbei können die verschiedenen Merkmale jeweils für sich oder in Kombination miteinander verwirklicht sein.

In den Abbildungen zeigen
- Fig. 1: eine vergleichende schematische Darstellung der Anordnung der zu untersuchenden Zelle bei der herkömmlichen Patch Clamp-Methode (A) und bei der Erfindung (B),
- Fig. 2: eine schematische Ausführung der Kapillare mit zu untersuchender Zelle bei der Erfindung,
- Fig. 3: die schematische Darstellung der Permeabilisierung auf jeweils einer Seite der Zellmembran bei der Erfindung,
- Fig. 4: zwei weitere schematische Ausführungen einer Kapillare mit zu untersuchender Zelle bei der Erfindung,
- Fig. 5: die schematische Darstellung einer erfindungsgemäßen Vorrichtung mit Kapillare und zu untersuchender Zelle mit Spülanschlüssen und Meßelektroden,
- Fig. 6: eine schematische Darstellung einer automatisierbaren erfindungsgemäßen Vorrichtung mit einer Vielzahl von Kapillaren (Einsaugen der Zellen),
- Fig. 7: eine schematische Darstellung einer weiteren automatisierbaren erfindungsgemäßen Vorrichtung mit einer Vielzahl von Kapillaren (Einzentrifugieren der Zellen),
- Fig. 8: Meßkurven beim Einbringen/Positionieren einer Zelle in eine Kapillare (Widerstandsmessung), und
- Fig. 9: fotographische Abbildung einer in die Spitze einer Glaspipette eingebrachten Zelle.

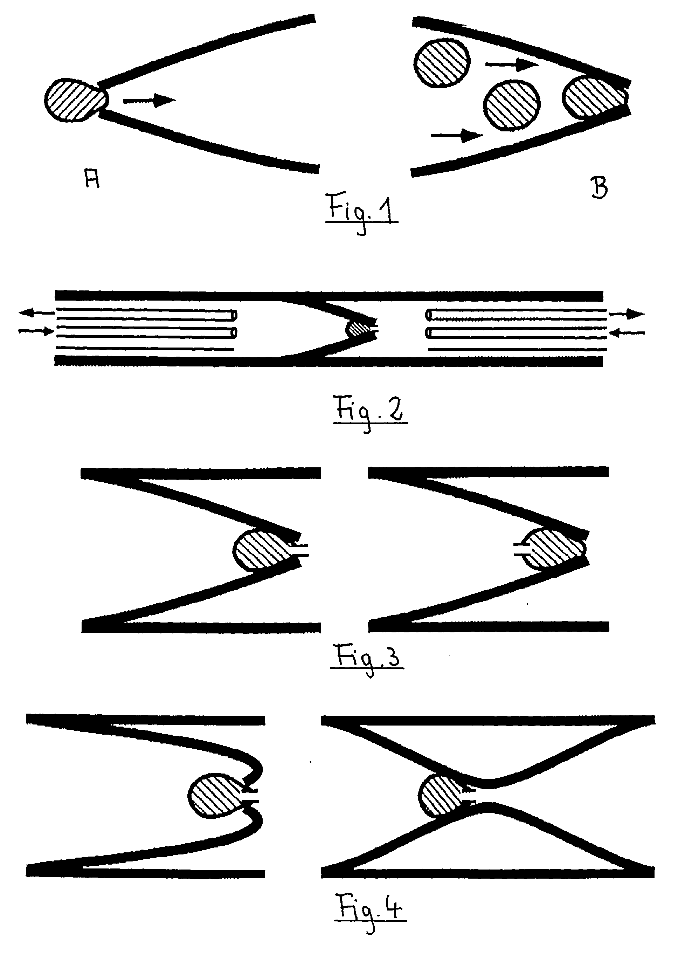

Fig. 1 dient zur Verdeutlichung des erfindungsgemäßen Prinzips gegenüber dem Stand der Technik. So zeigt Fig. 1 A schematisch, wie eine bei der herkömmlichen Patch Clamp-Methode zu untersuchende Zelle an der Spitze einer Mikropipette fixiert ist. Dabei wird zwischen dem Rand der Mikropipettenöffnung und der Zellmembran die notwendige innige Verbindung hergestellt. Der in Fig. 1 A gezeigte Pfeil symbolisiert die Ansaugrichtung, nachdem die Mikropipette unter Unterdruck mechanisch an die Zellmembran bewegt ist. Demgegenüber symbolisieren die beiden Pfeile in Fig. 1 B die Einbringrichtung der Zellen in eine Mikropipette bei der Erfindung, wenn die in Fig. 1 B beispielhaft dargestellten drei Zellen in die Mikropipette zum Beispiel als Suspension eingespült werden. Weiter zeigt Fig. 1 B, daß in dem sich verjüngenden Bereich der Mikropipette an deren Spitze eine Zelle mit der Innenfläche der Mikropipette (Kapillare) verklebt ist. An dieser so fixierten und positionierten Zelle kann die Patch Clamp-Untersuchung vorgenommen werden. Im Gegensatz zum Stand der Technik ist es nicht nötig, die Mikropipette auf die zu untersuchende Zelle zuzubewegen, sondern diese wird auf einfache Weise in die Pipette eingespült. Dieses Verfahren ist nicht nur einfach zu bewerkstelligen, sondern es kann auch auf die üblicherweise erforderliche mikroskopische Kontrolle und die Verwendung eines Mikromanipulators zum Verfahren der Pipette verzichtet werden. Dies gestattet in jedem Fall eine Miniaturisierung der Vorrichtung insgesamt.

In Fig. 2 ist schematisch eine Kapillare dargestellt, wie sie in dem erfindungsgemäßen Verfahren und bei der erfindungsgemäßen Vorrichtung verwendet werden kann. Die Kapillare ist als dünnes Röhrchen (Außendurchmesser 1,5 mm, Innendurchmesser 1,2 mm) ausgebildet, wobei im vorliegenden Fall etwa in der Mitte eine sich zu einer kleinen Öffnung verjüngende Verengung vorgesehen ist (engster Innendurchmesser 0,5 - 1 µm). An dieser Verengung ist bereits eine zu untersuchende Zelle (Durchmesser ca. 10 µm) positioniert und mit der Innenfläche der vorzugsweise aus Glas bestehenden Verengung verklebt. Die zwei kleinen Striche in der Öffnung der Verengung sollen eine Permeabilisierung der Zellmembran der fixierten Zelle zur rechten Seite hin symbolisieren. Weiter sind in Fig. 2 auf beiden Seiten der Verengung Zu- bzw. Ab-leitungen (Außendurchmesser 0,2 mm, Innendurchmesser 0,1 mm) für Suspensionen/Lösungen vorgesehen, wobei die jeweiligen Strömungsrichtungen durch links bzw. rechts angebrachte Pfeile symbolisiert sind. Diese Spüleinrichtungen können zum einen dem Einspülen der Zellsuspension selbst dienen (linke Zu- und Ableitung) oder zum Herausspülen der Zellsuspension, beispielsweise nach der Messung (linke Zu- und Ableitung). Zum anderen können sie zum Reinigen der Kapillare (linke und rechte Zu- und Ableitungen) oder zum Einbringen weiterer Substanzen wie pharmakologischer Substanzen, membranpermeabilisierender Substanzen und dergleichen (linke und rechte Zu- und Ableitungen) dienen. Außerdem sind Elektroden (z. B. aus AgCl-beschichtetem Silberdraht, Durchmesser 0,2 mm) in die Kapillare eingeführt, die der elektrischen Messung von Strömen und Spannungen sowie der Strominjektion dienen. Die Abmessungen der Kapillare, der Verengung und der Zu- und Ableitungen sind an die jeweiligen Erfordernisse anpaßbar, beispielsweise daran, welche Zellen welcher Größe untersucht werden.

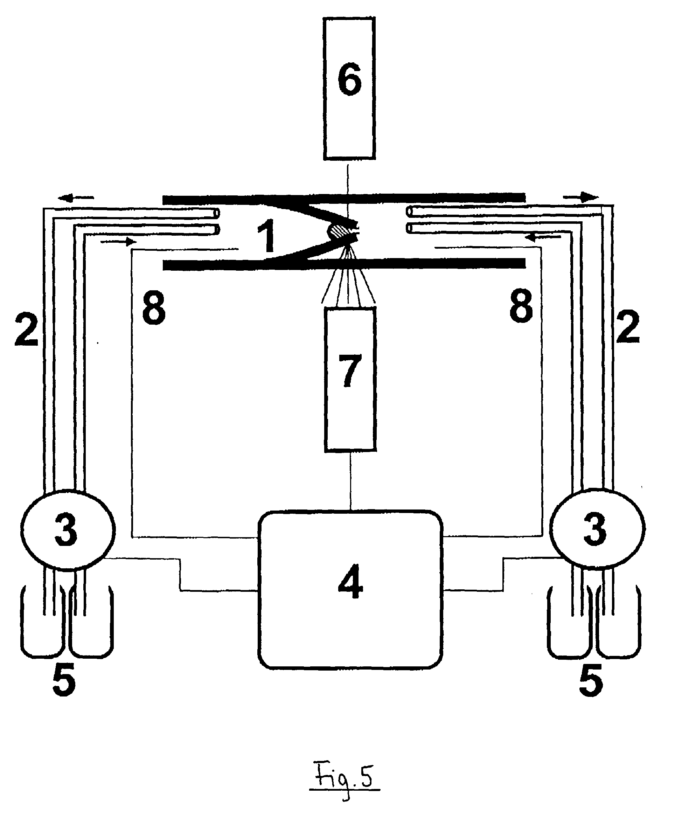

Fig. 3 zeigt in schematischer Darstellung, wie die Zellmembran einer zu untersuchenden Zelle entweder auf der Seite der Austrittsöffnung der Verengung oder auf der Seite der Eintrittsöffnung der Verengung permeabilisiert werden kann. Die Permeabilisierung ist dabei jeweils durch die Doppelstriche symbolisiert. Auf die entsprechenden Stellen in der Beschreibung wird Bezug genommen.

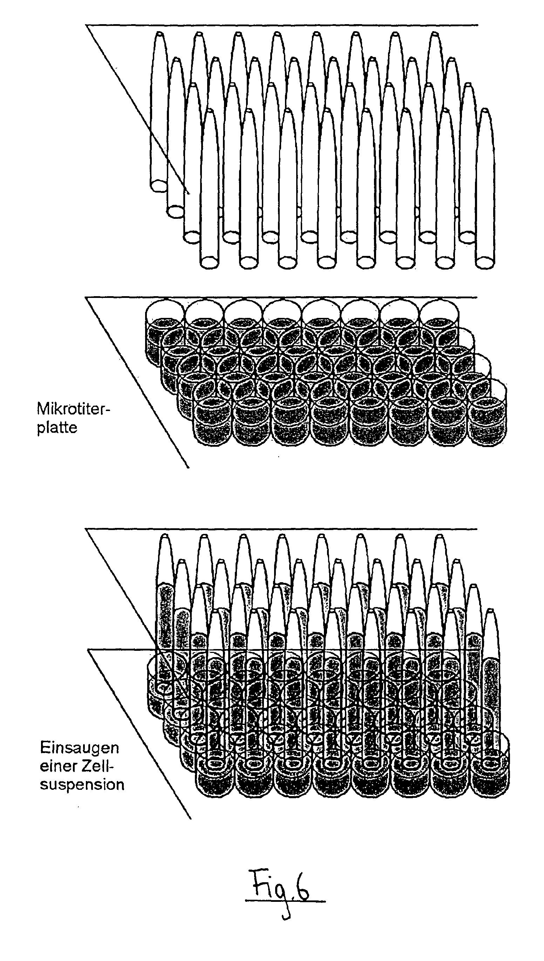

In Fig. 4 sind in schematischer Darstellung zwei weitere Konstruktionen von Kapillaren mit Verengungen dargestellt, wobei an den jeweiligen Verengungen eine zu untersuchende Zelle verklebt ist. In der linken Darstellung von Fig. 4 handelt es sich bei der Verengung um eine sich zu einer Austrittsöffnung verjüngende Engstelle, wobei die Austrittsöffnung als rückspringendes Rundloch ausgebildet ist. In der rechten Darstellung von Fig. 4 ist an der Kapillarwandung eine umlaufende Engstelle gebildet, an der die zu untersuchende Zelle in dem Bereich fixiert ist, in dem der Durchmesser der Kapillare auf den Wert des Durchmessers der Zelle abnimmt. Die Merkmale, die in Fig. 4 bei den beiden Ausführungen zu erkennen sind, werden hiermit ausdrücklich zum Inhalt der Beschreibung gemacht.

Eine erfindungsgemäße Vorrichtung ist in Fig. 5 schematisch dargestellt. Fig. 5 zeigt zunächst eine nach Art einer Mikropipette ausgebildete Glaskapillare 1, die im wesentlichen der Ausbildung von Fig. 2 entspricht. An der Verengung dieser Mikropipette 1 ist eine zu untersuchende Zelle positioniert.

Weiter sind Zu- und Ableitungen 2 dargestellt, die jeweils in die linke bzw. rechte Seite der Glaskapillare einmünden. In den Zu- und Ableitungen 2 sind Pumpen und gegebenenfalls Druckaufnehmer 3 angeordnet, die dazu dienen, Suspensionen oder Lösungen zu fördern bzw. den Flüssigkeitsdruck und/oder die Durchflußvolumina zu messen. Weiter sind in Fig. 5 Elektroden 8 dargestellt, die ebenfalls jeweils in das betreffende Ende der Glaskapillare 1 einmünden. Diese Elektroden finden sich übrigens auch in der Darstellung von Fig. 2 als entsprechende Striche unterhalb der Zu- und Ableitungen.

Weiter ist in Fig. 5 ein Laser 6 und ein zugehöriger Photodetektor 7 dargestellt, die dazu dienen, die Positionierung/ Fixierung der zu untersuchenden Zelle in der Glaskapillare 1 zu kontrollieren. Alle entsprechenden Bauteile sind weiter mit einem Datenverfassungs- und Steuergerät 4 verbunden, das die Spannungsklemme (Erläuterung siehe unten), Auswertung der Meßdaten und eine entsprechende Steuerung/ Regelung der Vorrichtung durchführt.

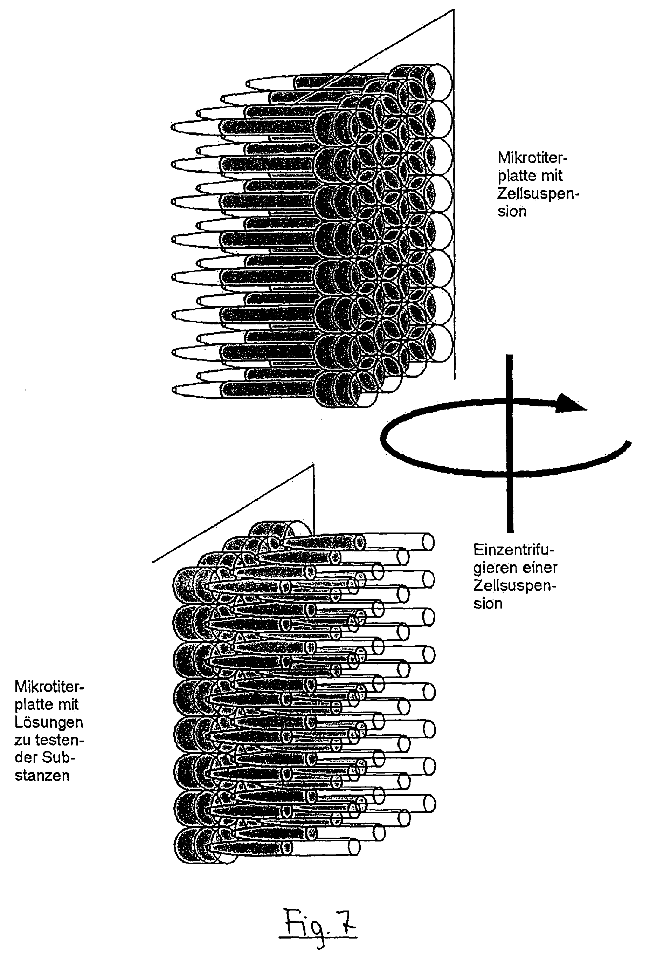

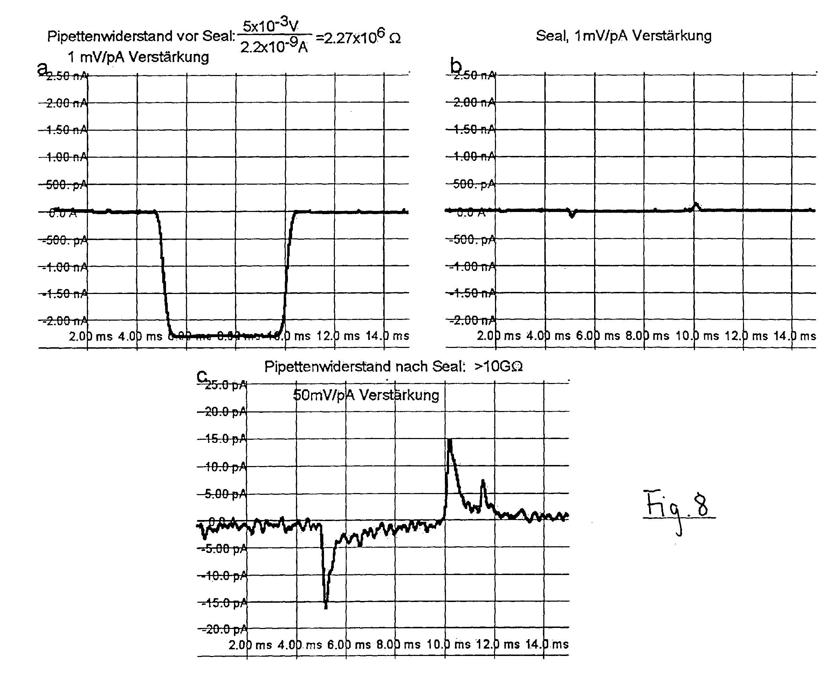

Die Fig. 6 und 7 zeigen schematisch Ausführungen der erfindungsgemäßen Vorrichtung, bei denen eine Vielzahl von als Glaspipetten ausgebildeten Kapillaren vorgesehen ist. Diese Glaspipetten sind in einer Ebene in Form eines regelmäßigen Rasters angeordnet und können von der Vorrichtung dann beispielsweise oberhalb einer der Anzahl der Pipetten entsprechenden Anzahl von Behältnissen angeordnet werden. Bei diesen Behältnissen kann es sich um die Vertiefungen einer Mikrotiterplatte handeln, wie dies in den Fig. 6 und 7 dargestellt ist. Die Pipetten sind dabei mit ihrer großen Öffnung in Richtung auf die entsprechenden Behältnisse ausrichtbar und können auf die Behältnisse zubewegt werden. Die Behältnisse, in diesem Fall die Vertiefungen der Mikrotiterplatte sind beispielsweise mit einer Suspension der zu untersuchenden Zelle oder mit verschiedenen Suspensionen solcher Zellen befüllt. Dann werden entweder, wie in der unteren Darstellung von Fig. 6 gezeigt, die Suspensionen mit Hilfe einer geeigneten Einrichtung aus der jeweiligen Vertiefungen der Mikrotiterplatten in die entsprechenden Pipetten eingesaugt, oder wie in Fig. 7 dargestellt einzentrifugiert. Dazu wird der Pipettenhalter an einem Rotor montiert, so daß durch Drehung eine Fliehkraft auf die Zellen in Richtung der Engstelle der Pipette ausgeübt wird. Zur Darstellung der Möglichkeit, bei der Erfindung Substanzen, z. B. pharmakologische Substanzen zu testen, ist in der unteren Abbildung von Fig. 7 eine weitere Mikrotiterplatte mit lösungen solcher Substanzen dargestellt, in die die mit Zellsuspension gefüllten Pipetten eintauchen.

### Versuch

Zunächst wurden Patch Clamp-Pipetten mit Hilfe eines digitalgesteuerten Pullers (Zeitz Instrumente, Augsburg) aus Borosilicat-Glaskapillaren (Clark Electromedical Instruments, Reading, Großbritannien) gezogen und durch Anschmelzen der Spitze (Polieren) auf einen Widerstand von 2 bis 3 Mega-Ohm (gemessen in wäßriger 150 mM Kochsalzlösung) gebracht. Zur Reduktion der elektrischen Kapazität bzw. des dielektrischen Rauschens wurden einige Pipettenspitzen mit Silikonkautschuk oder Paraffin überzogen.

Zur Messung wurde ein EPC 9 Patch Clamp-Verstärker (HEKA, Lambrecht) für Spannungsklemme und Datenakquisition verwandt. Die Daten wurden mit 10 kHz digitalisiert und bei 2 kHz gefiltert. Es wurde ebenfalls eine Software der Firma HEKA für die Analyse verwendet. Unter "Spannungsklemme" ist hier eine übliche Schaltungstechnik für die Kontrolle elektrischer Parameter bei Patch Clamp-Messungen zu verstehen. Eine Rückkopplungsschaltung über einen extrem hohen Widerstand speist gerade so viel Strom in das biologische Präparat, daß die gemessene Spannung auf einem vorgewählten Wert verbleibt. Der analoge Rückkopplungskreis besitzt eine hohe Zeitauflösung und erlaubt, mit verschiedenen Kompensationstechniken kapazitive Ströme, Spannungsabfall an Serienwiderständen sowie Leckströme zu unterscheiden von den Ionenströmen, die durch die untersuchten Membranstrukturen fließen.

Anschließend wurde die Pipette mittels eines AgCl-beschichteten Silberdrahtes an den Vorverstärker angeschlossen und mit einer modifizierten Ringerlösung gefüllt, die 145 mM NaCl, 5 mM KCl, 2 mM CaCl₂, 1 mM MgCl₂, 10 mM Glukose und 10 mM HEPES enthielt (pH 7,4). Anschließend wurden 100 - 500 Zellen (Jurkat T Zellen, ATCC, VA, USA) mit einem feinen Glasröhrchen in die Pipette eingebracht. Die Pipette wurde in ein Bad mit Gegenelektrode eingetaucht. Ein Spannungspuls von 5 mV erlaubte die Messung des elektrischen Widerstandes (siehe Fig. 8a). Durch Applikation eines Druckes von 0,5 bar wurden dann Zellen in Richtung der Pipettenspitze gespült und der Druck rasch entfernt, als der elektrische Widerstand der Pipette durch eine Zelle in der Pipettenspitze zunahm (Fig. 8 b).

Mit diesem Versuch konnten Gigaseals erzeugt werden, deren elektrischer Widerstand (wie bei der herkömmlichen Patch Clamp-Methode) im Bereich von > 10 Gigaohm lag (siehe Fig. 8 c).

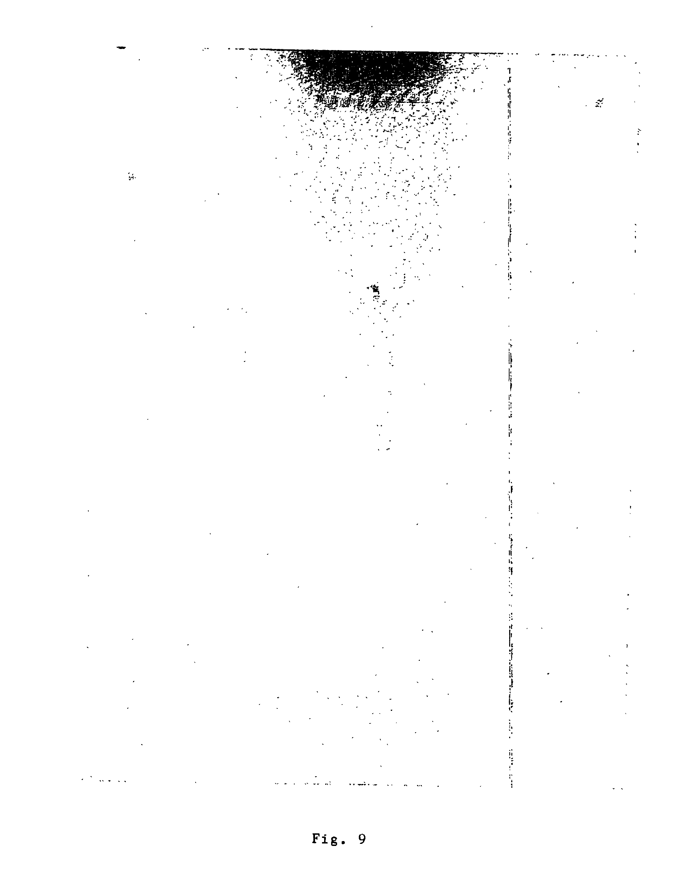

Bei dem Versuch konnte weiter gezeigt werden, daß die zu untersuchende Zelle beim Versuch in der Spitze der Glaspipette positioniert war. Dies ist in Fig. 9 anhand einer Fotografie dargestellt. Weiter wurde im Versuch gezeigt, daß durch die Applikation eines zusätzlichen kurzen Druckpulses (200 ms, 1 bar) oder eines elektrischen Pulses (500 mV, 0,1 ms) die Zellmembran einseitig perforiert werden konnte. Dies war an der Zunahme des kapazitiven Ladestroms erkennbar. Der Gigaseal war häufig über lange Zeiträume (10 - > 60 min.) stabil und blieb sogar bei gezielter mechanischer Erschütterung der Mikropipette erhalten.

Der beschriebene Versuch zeigt somit eindeutig, daß durch Einbringen einer Zelle in eine Kapillare aus Glas mit einer Verengung, insbesondere eine sich verjüngende Glaspipette eine ausreichend dichte Verbindung zwischen Zellmembran und Pipettenwand und damit die genannten Vorteile erreicht werden können.

## Patentansprüche

1. Verfahren zur Untersuchung von Zellen oder dergleichen mit Hilfe der Patch Clamp-Methode, wobei mindestens eine Zelle in das Innere einer Kapillare, die auf ihrer Länge mindestens eine Verengung mit einem Durchmesser, der kleiner als der Durchmesser einer Zelle ist, aufweist, eingebracht und dort unter Ausbildung eines ausreichend dichten Kontaktes zwischen Zellmembran und Kapillarinnenfläche positioniert wird und anschließend an der derart positionierten Zelle die Patch Clamp-Untersuchung vorgenommen wird.

2. Verfahren nach Anspruch 1, **dadurch gekennzeichnet, daß** das Einbringen und Positionieren der Zelle in die Kapillare durch Einspülen oder Einsaugen einer Suspension oder Lösung, die die Zelle enthält, erfolgt.

3. Verfahren nach Anspruch 1 oder Anspruch 2, **dadurch gekennzeichnet, daß** das Einbringen und Positionieren der Zelle in die Kapillare durch Einzentrifugieren einer Suspension oder Lösung, die die Zelle enthält, erfolgt.

4. Verfahren nach einem der vorhergehenden Ansprüche, **dadurch gekennzeichnet, daß** es sich bei der Kapillare um eine Glaskapillare handelt.

5. Verfahren nach einem der vorhergehenden Ansprüche, **dadurch gekennzeichnet, daß** es sich bei der Kapillare um eine sogenannte Mikropipette handelt.

6. Verfahren nach einem der vorhergehenden Ansprüche, **dadurch gekennzeichnet, daß** die Positionierung der Zelle im Kapillarinneren vor Durchführung der Patch Clamp-Untersuchung kontrolliert wird, vorzugsweise durch Druckmessung oder Durchflußmessung oder Messung des elektrischen Widerstands beim Einbringen der Zelle in das Kapillarinnere oder durch Auswertung optischer Signale, insbesondere mit Hilfe eines Lasers, nach dem Einbringen der Zelle in das Kapillarinnere.

7. Verfahren nach einem der vorhergehenden Ansprüche, **dadurch gekennzeichnet, daß** die Zelle nach Durchführung der Patch Clamp-Untersuchung aus der Kapillare entfernt und die Kapillare, vorzugsweise durch Spülung mit geeigneten Lösungsmitteln und/oder Chemikalien, gereinigt wird.

8. Vorrichtung zur Untersuchung von Zellen oder dergleichen mit Hilfe der Patch Clamp-Methode, insbesondere zur Durchführung des Verfahrens nach einem der vorhergehenden Ansprüche, **gekennzeichnet durch**
- mindestens eine Kapillare, die auf ihrer Länge mindestens eine Verengung mit einem Durchmesser, der kleiner als der Durchmesser einer Zelle ist, aufweist, und die dazu vorgesehen ist, daß in ihr Inneres eine Zelle eingebracht und dort unter Ausbildung eines ausreichend dichten Kontaktes zwischen Zellmembran und Kapillarinnenfläche positioniert wird,
- mindestens eine Einrichtung zum Einbringen einer Zelle in das Kapillarinnere und zur Positionierung der Zelle im Kapillarinneren, und
- gegebenenfalls weitere übliche Bauteile einer Vorrichtung zur Durchführung der Patch Clamp-Methode.

9. Vorrichtung nach Anspruch 8, **dadurch gekennzeichnet, daß** die Einbring- und Positioniereinrichtung zum Einspülen oder Einsaugen mindestens einer Suspension oder Lösung in das Kapillarinnere ausgebildet ist.

10. Vorrichtung nach Anspruch 9, **dadurch gekennzeichnet, daß** vorgesehen sind:
- Behältnisse zur Aufnahme der Suspensionen oder Lösungen,
- Zuleitungen und/oder Ableitungen zwischen den Behältnissen und der Kapillare,
- Pumpeinrichtungen für die Suspensionen oder Lösungen, sowie gegebenenfalls
- Einrichtungen zum Messen von Drücken und Durchflußvolumina.

11. Vorrichtung nach einem der Ansprüche 8 bis 10, **dadurch gekennzeichnet, daß** die Einbring- und Positioniereinrichtung zum Einzentrifugieren mindestens einer Suspension oder Lösung in das Kapillarinnere ausgebildet ist.

12. Vorrichtung nach einem der Ansprüche 8 bis 11, **dadurch gekennzeichnet, daß** es sich bei der Kapillare um eine Glaskapillare handelt.

13. Vorrichtung nach einem der Ansprüche 8 bis 12, **dadurch gekennzeichnet, daß** es sich bei der Kapillare um eine sogenannte Mikropipette handelt.

14. Vorrichtung nach einem der Ansprüche 8 bis 13, weiter **gekennzeichnet durch** Einrichtungen zur Messung des elektrischen Widerstandes beim Einbringen der Zellen in das Kapillarinnere oder Einrichtungen zur Auswertung von optischen Signalen, vorzugsweise Laserlichtsignalen, nach dem Einbringen der Zellen in das Kapillarinnere.

15. Vorrichtung nach einem der Ansprüche 8 bis 14, insbesondere zur Untersuchung von Zellen oder dergleichen mit Hilfe einer automatisierten Patch Clamp-Methode, **dadurch gekennzeichnet, daß** eine Vielzahl von in Anspruch 8 definierten Kapillaren, insbesondere von Mikropipetten, vorzugsweise in einer regelmäßigen flächigen Anordnung vorgesehen sind.

16. Vorrichtung nach Anspruch 15, **dadurch gekennzeichnet, daß** eine Vielzahl von Behältnissen, insbesondere Mikroküvetten, für die Aufnahme von Suspensionen oder Lösungen, die Zellen oder dergleichen enthalten, vorgesehen ist, wobei vorzugsweise die Anzahl an Behältnissen der Anzahl an Kapillaren entspricht.

17. Vorrichtung nach Anspruch 16, **dadurch gekennzeichnet, daß** die Kapillaren und die Behältnisse derart zueinander angeordnet oder zueinander beweglich sind, daß die Suspensionen oder Lösungen und damit die in ihnen enthaltenen Zellen oder Substanzen in das Innere der Kapillaren überführbar sind.

18. Verwendung einer Kapillare, vorzugsweise einer Glaskapillare, insbesondere einer Mikropipette, die auf ihrer Länge mindestens eine Verengung mit einem Durchmesser, der kleiner als der Durchmesser einer Zelle ist, aufweist, zur Durchführung des Verfahrens nach einem der Ansprüche 1 bis 7.
